# EUROPEAN PATENT APPLICATION

(11) **EP 1 591 775 A1**
(43) Date of publication of application: **02.11.2005**
(21) Application number: 05103406.4
(22) Date of filing: 26.04.2005
(51) Int. Cl.: G01N 27/07, G01N 27/10, G01N 27/04, G01N 33/28

(54) **Sensor for the detection of water in fuel and system for the interruption of fuel delivery comprising said sensor**

(30) Priority: 27.04.2004 IT MI20040188; 27.04.2004 IT MI20040818
(71) Applicant: Pregnolato, Aldo Stefano, 20133 Milano (IT)
(72) Inventor: Pregnolato, Aldo Stefano, 20133 Milano (IT)
(74) Representative: Faggioni, Carlo Maria

(57) **Abstract**

A sensor and corresponding system for the detection of water in a tube for the delivery of di-electric fluids is disclosed, comprising a pair of opposite conductive surfaces (F1,F2) provided with holes between which surfaces a preset electric voltage is established, the two surfaces being mutually insulated and distanced by such a measure (M) that, at the delivery rate, the flow of water droplets short-circuits at least partly said conductive surfaces (F1,F2) provided with holes, generating a flow of detectable electric current.

## Description

### Field of the invention

The present invention relates to a sensor for the detection of water in dielectric fluids, in particular liquid fuels, and to a system for delivery cut-off of said fluids upon water detection employing said sensor or probe.

### Background Art

As known, water is the most common compound on the earth surface and has, in all its variants provided by the three isotopes of hydrogen and oxygen, remarkable properties as a class 2 (liquid) electric conductor.

Petrol instead is a neutral-reaction, light mixture of hydrocarbons, totally devoid of water as well as of impurities and evaporation residues. Diesel oil is a high-boiling oil fraction which is obtained by fractional distillation of the heavy oils derived from primary oil distillation. Both fuels do not contain water, except biodiesel oil only, or "white diesel", which, as we shall see in the following, contains suitably treated water. Due to their composition and features, the above-mentioned fuels behave as insulators.

The delivery of petrols, diesel oils and fuels in general, in particular for motor propulsion, is unable to either detect or prevent or manage the entry of water due to seepage into the tank or fuel lorries from which the same fuel is taken.

A number of vehicles report malfunctioning and damages due to the introduction of even a minimal quantity of water, despite the adoption of suitable filters.

In biodiesel oils it is precisely the water which allows to improve combustion, also reducing particle production, and it is again water which allows to reduce carbon dioxide and to decrease combustion temperatures reducing the production of nitrogen oxides.

The above-detailed balance is achieved through stabilising additives which are capable of mixing water and diesel oil in the right proportions and ways. It must be remembered that the water introduced in this particular process, without considering the additives, is treated in order to reduce its conductivity to a maximum of 30 microS/cm, compared to over 1,500 microS/cm of normal drinking water.

This balance, however, is suddenly impaired if water foreign to the preparation is added.

It is hence important for any fuel to prevent quantities of water, even traces thereof, from accidentally entering the fuel tank.

For such purpose a system is hereby proposed, which is arranged in the tank inlet tube and is capable of detecting in real time the presence of water traces in the delivered insulating liquid and hence also of alerting the user before excessive quantities of water are introduced in the tank.

An embryo system for water detection upon fuel delivery is set forth by the same inventor in Italian patent no. 1,290,853. In this patent the concept of using a probe equipped with opposing cathodes is disclosed, intended to detect the dielectric coefficient of the liquid or emulsion passing through. Such system further needs a comparison table, through which the logical unit of the system operates to establish the type and quality of the liquid being detected.

However, it has been noticed that this system as described does not work correctly for the task sought in this application: i.e., in particular, it is unable to supply detailed, prompt and reliable information on the presence of water traces, for example in the hydrocarbons introduced in a tank.

### Summary of the invention

It is an object of the present invention to provide a sensor and a corresponding detection system which is extremely efficient in detecting water traces in the di-electric liquid and which allows to promptly cut off delivery of the same to a tank.

Such object is achieved through a device as described in its essential features in the accompanying main claim.

Other inventive aspects of the device are described in the dependent claims.

The device, by the electric principle, detects in real time during refuelling the presence of water or partially water-containing emulsions in any percentage, due to the arrangement and the possible settings of the sensor used and of the associated system, introduced in tanks during the delivery of petrols, diesel oils and insulating liquids in general.

The operating principle of the system subject of the present application is based on the development of a particular probe/sensor, suitably connected to respective electronics, apt to produce, exclusively in the presence of water, weak electric signals for sound and/or visual signalling upon water introduction and the possible registration of the event in a suitable unit.

Hence, according to a first aspect of the invention, the Applicant has developed an improved version of the known detection probe, providing a sensor equipped with opposite fine-mesh metal cathodes, which makes it possible to signal with the highest sensitivity and precision in real time during delivery, regardless of the water percentage present and of the flow rate considered.

According to another aspect, sensor sensitivity can be adjusted, both mechanically and electronically.

The electric signals generated by the sensor of the invention can be used within the complex system, wherein at need the accessory devices are driven, such as for example a further sensor or sensor system for water detection within the tank and/or opening and closure valves to interrupt refuelling in case it is not interrupted by the user, and/or to divert said "contaminated" refuelling outwards or to a containment area different from the tank, thereby also preventing any minor contamination of the fuel therein contained.

According to a last aspect, the above system can be integrated with other devices subject of separate applications by the same Applicant, such as for example the device described in Italian application no. MI2004A000818 for water detection and management within the considered tank, the device described in Italian application no. MI2004U000293 concerning management of the fuel quantity together with water detection, as well as the system described in Italian application no. MI2004A000976 concerning a system for signalling the erroneous delivery of petrol to diesel-powered vehicles.

### Brief description of the drawings

Further features and advantages of the device according to the invention will in any case be more evident from the following detailed description, given by way of example and illustrated in the accompanying drawings, wherein:
figs. 1A and 1B are side elevation and top views, respectively, of a first embodiment of a plate of the sensor according to the invention, a partial enlargement of which is shown in fig. 1C;
figs. 2A and 2B are side elevation and top views, respectively, of a second embodiment of a plate of the sensor according to the invention, a partial enlargement of which is shown in fig. 2C;
fig. 3 is an exploded view of a detection device equipped with the sensor of the invention;
fig. 4 is a section view of the device of fig. 3; and
fig. 5 is a diagrammatic view of a tank equipped with the sensor of the invention and with an additional system with a sensor on the bottom and valves, according to a possible embodiment.

### Detailed description of a currently preferred embodiment

The system subject of the application consists of a device for the detection of water in real time and of a further unit for the confinement of the water accidentally introduced, during the delivery of petrols, diesel oils and isolating liquids in general, to the corresponding tanks or fuel trucks.

The detection device can be manufactured separately and installed later on a fuel inlet manifold, interrupting continuity thereof, or it can be tailor-designed to be manufactured as an integral part of the tank itself.

Once the detection has occurred, the system provides to immediately inform the operator through sound and/or visual signals, said operator can thereupon immediately stop refuelling preventing contamination of the liquid contained in the tank.

It is further possible to provide an additional sensor system for the detection of water within the tank and/or a valve system which provides to the automatic refuelling stopping, should the operator not do so, and to the confinement of the tiny quantity of fuel mixed with water in an area different from the tank, as described below.

For the detection of water the system exploits a sensor of the type used in the accompanying drawings, of a varying diameter and geometry, according to the inlet tube and flow rate being considered. The sensor illustrated here by way of example refers to deliveries to motorvehicles powered by unleaded petrol and diesel oil, which vary from 0 to 50 litres per minute. Such flow rates, however, can exceed 200 It./minute in case of dispensers for fuel trucks and lorries. For this reason the sensor subject of the application has a varying diameter and geometry (for example, it can be non planar) according to reference flow rates; however, its basic arrangement, together with the operating principle, is always as described in the following.

The sensor according to the invention substantially consists of two plates provided with holes, of a conductive material, mutually opposite and insulated, forming conducting cathodes. Alternatively, a single plate provided with holes can be provided with the two opposite sides thereof coated in a conductive material so as to be mutually insulated; or two mutually opposite nettings, with varying mesh and thickness, kept sufficiently distant from each other so as not to be in electric contact in normal operating conditions.

The distance between the two cathodes and the shape of the plate holes or mesh is such that, at the flow rates considered and with the water percentage that the manufacturer wants to intercept, the passage of small water droplets within a di-electric liquid puts into communication the two sensor sides determining the flow of an electric current, which is detected by a suitable electronic control device connected to the two cathodes.

The mutual distance between the two plates or nettings forming the cathodes is set and established mechanically according to the operating conditions in which the sensor is required to work (for example according to flow rate, treated di-electric liquid, desired sensitivity, and so on). In particular, the upper limit of such distance is determined by the need for a continuous mass of water (for example a droplet) to touch both plates at the same time: typically, such maximum distance can be of 2-3 mm. The minimum distance is instead that necessary to prevent short-circuit between the two plates even in the absence of water.

According to a different embodiment, the two plates are mounted on an adjustable frame or support, so that the distance can be varied at will by the user according to the conditions of use, for example according to mounting diagrams provided by the manufacturer himself.

Sensor size and geometry also, as well as number and features of the holes punched therein, are established according to operating conditions, for example according to empirical tests. This allows to vary the physical ability of the sensor to recognise the presence of water or emulsions thereof.

Other sensitivity adjustments can be carried out electronically by setting a threshold signal, so as to down-adjust the sensitivity of the sensor used, regardless of the physical features thereof.

Design restraints are also caused by the need to ensure a desired delivery flow rate, i.e. avoiding that the probe/sensor introduces an excessive flow resistance which would cause an undesired counterpressure originating backups of liquid in the tank inlet tube.

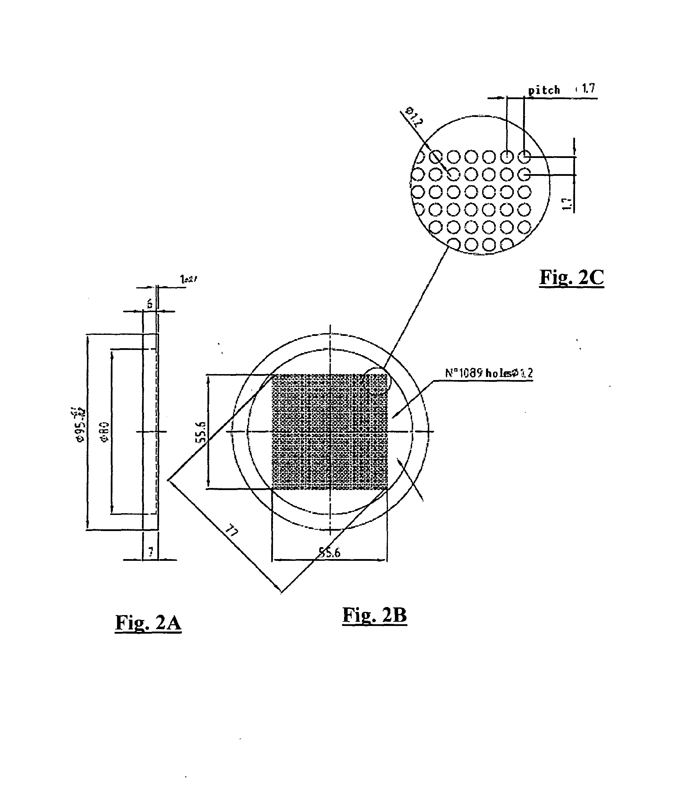

In the example shown in fig. 2, the two plates are circular and the useful portion of the sensor has a square shape with a side measuring about 55 mm, the pitch between the holes being of 1.7 mm and their diameter of 1.2 mm, adding up to 1,189 holes. In the following table the speeds are reported, which are obtained according to the number and size of the holes with a flow rate of 50 1t./min.

| **DELIVERY FLOW RATE (litres per minute)** | **NO. OF HOLES** | **DIAMETER (mm)** | **PITCH (mm)** | **OUTPUT SPEED (metres per second)** |
|---|---|---|---|---|
| 50 | 1.189 | 1.2 | 1.7 | 0.68 |
| 50 | 700 | 1.2 | 1.7 | 1.05 |
| 50 | 400 | 1.2 | 1.7 | 1.84 |
| 50 | 200 | 1.2 | 1.7 | 3.58 |
| 50 | 300 | 1.5 | 1.7 | 1.57 |
| 50 | 309 | 1.2 | 2 | 2.38 |
| 50 | 309 | 1 | 2 | 3.43 |
| 50 | 250 | 1.2 | 2 | 2.95 |
| 50 | 350 | 1 | 2 | 3.03 |

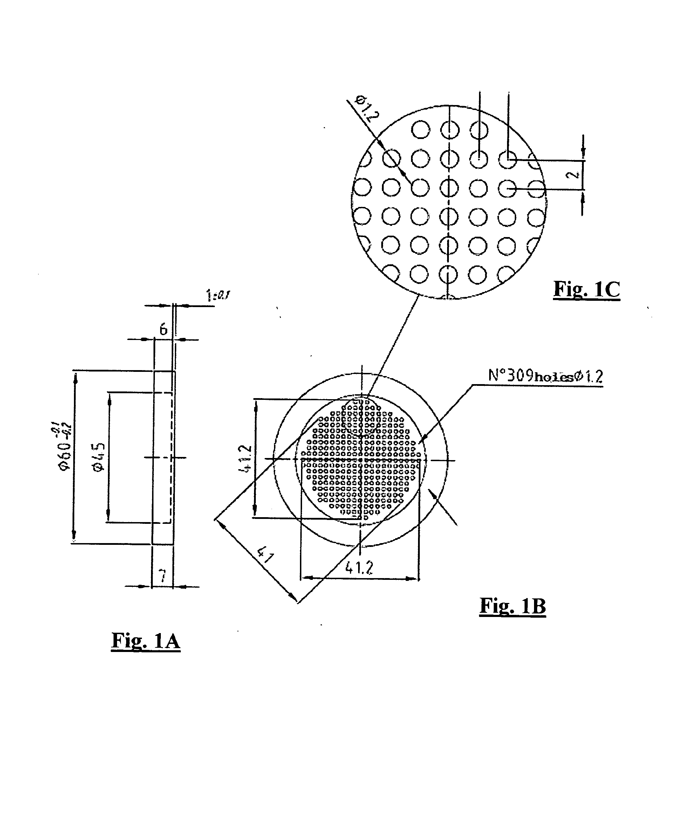

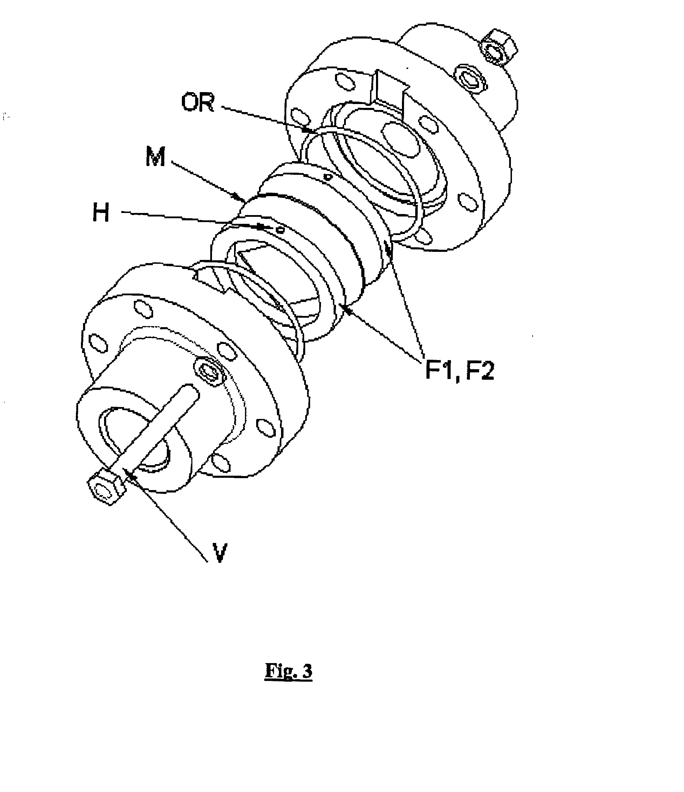

The plates, shaped as shown by way of example in fig. 1 or fig. 2, are then coupled and assembled to form the sensitive part of the device, an exploded view of which is shown in fig. 3.

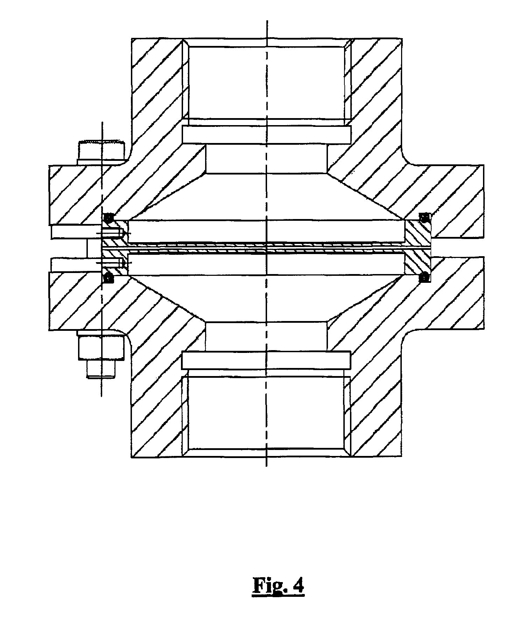

The detection device according to the invention, as shown in figs. 3 and 4, comprises two half-portions A and B which may be coupled, between which the proper sensor part is enclosed (in sequence: insulating O-rings OR, plates F1 and F2, and a di-electric membrane M). The two half-portions A and B, assembled for example through screws V, end externally with tube-like ports for attachment to the manifold of the tank considered.

The entire device is then mounted to the desired delivery manifold of the di-electric liquid, for example in the inlet tube of a motorvehicle petrol tank.

The two sides of the probe are mutually parallel but, depending on how they are mounted in the detection device or on how the latter is installed in the inlet tube, they can lie at a certain angle to the entry direction of the liquid being considered.

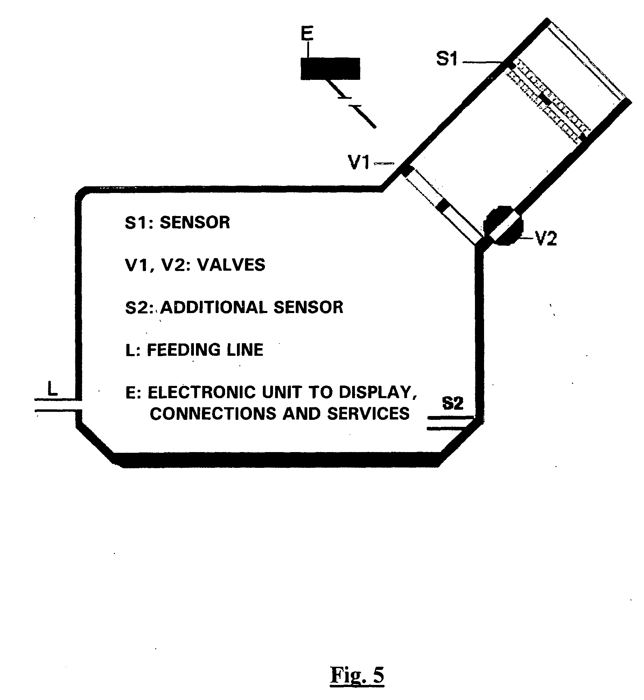

In the case of two sheets of a conductive material, provided with holes, rigid and with an empty gap between them (S1 in fig. 5), the position of the holes between the sheets can coincide or be offset, depending on the sensitivity that one wants to impart the system.

In the example shown in fig. 3, between the two plates F1 and F2 of conductive material (for example copper, conductivity 57x10⁶ S/m), there is sandwiched a plate of an insulating material M having a thickness of 2 mm. The two plates of conductive material F1 and F2 have peripheral holes H for the passage of conductive, perfectly insulated wires, embedded or not in the insulating material itself. Said conductive wires H have the function of transferring electric signals to and from the sensor plates provided with holes.

The plate of insulating material M lies so that the two sides of a conductive material are perfectly insulated from each other and so as to avoid that the delivery pressure causes the sides to flex and/or touch each other. The thickness of the insulating material or in any case the distance between the sides of the plates or between the two nettings used, can of course vary - as already mentioned above - according to the sensitivity desired and to the flow rates being considered.

The insulating material used in the sensor must be compatible with the use described and the environment being considered, such as for example fluoridated rubbers (Teflon) or high-density thermoplastic materials, such as polyethylene and polythene.

The operation of the device provides that, when water occurs in the fuel delivered, even in infinitesimal quantities, the two sides of conductive material of the sensor, powered with low voltage, generate a micro-current for the entire time in which they are interested by water, during delivery. The sensor of the invention is hence able to detect how many drops, or continuous portions thereof, pass through the plates provided with holes in the time unit, counting the electric impulses, at a processing speed which is predetermined in the electronic control device.

The superficial water tension, which is higher than in the fuels considered, in the case of the sensor considered does not represent an actual problem, both because a suitable distance is established between the two sides of conductive material (which distance is occupied or not by insulating material), and because the delivery flow rate makes sensitivity to capillarity phenomena unlikely, which phenomena depend on superficial tension.

As mentioned before, similarly to the main sensor mounted in the loading manifold, it is possible to provide one or more further sensors S2 (fig. 5) on the bottom of the tank, consisting of two walls of a conductive material, mutually insulated and arranged at such a mutual distance as not to be affected by the effects of superficial tension, for the detection of the water introduced or present due to normal condensation even on the bottom of the tank being considered. Water does not emulsify with fuel and sediments on the bottom of the tank, therefore by arranging such sensor in the upper part of a low point of the tank being considered, which is naturally there or purpose-built, away from the fuel feeding line, once such "water containment" area is full it is possible to warn the user and proceed to bleeding, manually or through a further valve.

Moreover, once the water has been detected during the delivery step, it is possible to actuate an optional closure valve V1 (fig. 5) of the tube being considered, so as to stop inflow of further fluid in the tank. A containment chamber for the tiny amount of fuel mixed with water or for water only is thereby obtained, which is defined between the mouth of the manifold and the insulation valve V1. In this case it is possible to provide a further passage port equipped with a controllable valve V2 to deviate this tiny amount of fuel mixed with water to a dedicated area, in any case outside the tank. Drain valve V2 can be timed and be allowed to open after closure of V1.

Further interventions for automatic adjustments can be provided during the design step and be introduced in the electronic control unit E.

Electronic control unit E handles the management of the signals coming from the sensor and the actuation of the warnings, in addition to any management of the valve system described.

In order to avoid any possible overcurrent danger, in case of system faults it is provided that it is cut out when a power consumption load slightly higher than the normal working consumption is reached.

The entire system can be set up for connection to a GPS or GSM interface, so that certain event-specific data may be recorded for insurance and other legally provided purposes.

The advantages of the system described appear to be evident even by only adopting the detection device and the corresponding warning system. In the automotive use, for example, said system not only eliminates the likelihood of damage due to water entry, by signalling inflow thereof and being able to promptly stop refuelling, but it also prevents the vehicle considered from being workshop-bound for the maintenance operations required in case of water penetrating in the engine.

The system set forth above can also be used advantageously for all the fuel trucks and tanks containing insulating liquids and emulsions (oils, valuable liquids, etc.), wherein it can instead be useful to detect, warn of and/or stop the presence of water or of partly watery emulsions.

However, it is understood that protection of the invention described above is not limited to the particular embodiment illustrated, but extends to any other equivalent construction variant which falls within the scope of the attached claims.

## Claims

1. A sensor for the detection of water in a delivery tube for di-electric fluids, **characterised in that** it comprises a couple of opposite conductive surfaces provided with holes, between which surfaces a preset electric voltage is established, the two surfaces being mutually insulated and distanced by a measure so that, at the delivery rate provided, the flow of water droplets short-circuits at least partly said conductive surfaces provided with holes, generating a flow of detectable electric current.

2. The sensor as in claim 1), wherein said conductive surfaces are in the form of metal plates provided with holes.

3. The sensor as in claim 2), wherein said plates provided with holes have a hole density between 11 and 40 holes/cm².

4. The sensor as in claim 1), wherein said conductive surfaces are in the form of metal nettings.

5. The sensor as in any one of the previous claims, wherein said surfaces provided with holes are arranged at a distance of 1-3 mm.

6. The sensor as in any one of the previous claims, wherein said opposite surfaces provided with holes are separated by insulating material.

7. The sensor as in claim 6), wherein said conductive surfaces are obtained by metal coating the two sides of a di-electric plate provided with holes.

8. A system for the detection of water, and consequent stopping, during the delivery of substantially insulating liquids, such as fuels, to a tank, **characterised in that** it comprises a sensor such as in any one of the previous claims, associated with a logical control unit apt to detect the presence of electric current within the sensor and to issue a corresponding signal to a warning device or to a device actuating cut-out valves.

9. The system as in claim 8), wherein said cut-out valves consist of valves to automatically stop said delivery and the subsequent discharge into the outer environment of the water detected.

10. The system as in claims 8) or 9), wherein said sensor is arranged in an inlet manifold in a motorvehicle tank.

11. The system according to one or more of claims 8) to 10), further comprising one or more additional sensors for the detection of water on the bottom of the tank being considered, such water being confined to the bottom of said tank in a naturally occurring or purpose-built area.
